Europäisches Patentamt

**European Patent Office**

**Office européen des brevets**

(19)

(11) Publication number: **0 059 328**

**A1**

(12) # EUROPEAN PATENT APPLICATION

(21) Application number: **82100768.9**

(22) Date of filing: **03.02.82**

(51) Int. Cl.³: **A 61 B 5/00**
**G 01 K 11/16**

(30) Priority: **03.02.81 IT 950381 U**

(43) Date of publication of application:
**08.09.82 Bulletin 82/36**

(84) Designated Contracting States:
**AT BE CH DE FR GB IT LI LU NL SE**

(71) Applicant: **Cassina, Enrica**
**Via Moscova 40/8**
**I-20121 Milano(IT)**

(72) Inventor: **Cassina, Enrica**
**Via Moscova 40/8**
**I-20121 Milano(IT)**

(74) Representative: **Riccardi, Sergio**
**Riccardi & Co. Via Macedonio Melloni, 32**
**I-20129 Milano(IT)**

(54) **Microencapsulated liquid crystals plate for mammary thermographic detection.**

(57) A plate for mammary thermographic detection based on microencapsulated liquid crystals, comprising a flexible support (1) having two areas (2, 3) of anatomic shape, provided with said liquid crystals, covering all quadrants of the breast, including the outer ones (4), the plate being preferably provided with a room temperature indicator (10; 14, 15, 16) and with means to hold it adhering to the entire breast shape, so as to allow a correct detection and instant comparison of the state of both mammae.

Fig. 2

EP 0 059 328 A1

- 1 -

"Microencapsulated liquid crystals plate for mammary thermographic detection"

The present invention relates to a plate for mammary thermographic detection, having considerable features of utility and ease of use.

Since about a decade the technique of mammary thermographic detection was introduced, using microencapsulated liquid crystals to show abnormal or pathological states of the breast and this technique is now very well known and widely described in the literature.

Thermographic detection is effected by rigid or flexible plates having areas with microencapsulated liquid crystals and flexible plates were recently put on the market, which are applied on the breast and through two wings are held in position under the axillae, while the liquid crystal thermosensitive layers are forming two rectangular areas to be leaned on the breast.

These plates have however several drawbacks in practicala use, as the patient has to hold firmly and precisely the plate on the breast, and the rectangular areas provided with liquid crystals have neither the form nor the extent adapted to allow contemporaneous examination of the symmetrical zones of the breast, as it is necessary to carry out an equality comparison between the thermographic maps of both mammae, and more particularly to carry out the contemporaneous detection on the outer quadrants of the breast, which are the most important points to be checked, as it is just these outer quadrants where there is the highest likelihood of abnormal or pathological states. Finally the patient has no possibility to check if the room temperature is in the proper range to effect the detection.

All these drawbacks are removed by the plate for mammary thermographic detection being the subject matter of the present invention and consisting of a flexible support having two areas of anatomic shape, provided with layers of thermosensitive liquid crystals, and covering totally and contemporaneously all the mammary quadrants, including the outer ones.

The thermosensitive areas may have two or more superposed layers of liquid crystals, which are changing colour at different temperature so as to allow a broader range of temperatures for detection while for a more accurate, professional survey of the thermographic map it is also possible to provide several plates, each provided with one layer of liquid crystals changing colour at a predetermined temperature.

To the plate one may apply a strap to be worn on shoulders or on the neck, or a ribbon starting from the two lateral ends of the plate and to be closed on the back like the usual brassières, or both strap and ribbon may be provided together on the same plate. At last the plate may also have a cord keeping the lower central portion of the plate adhering to the body, which is also tied on the back.

Furthermore the plate according to the present invention preferably has a little room temperature indicator, also made of liquid crystals, applied on the support to check if the room temperature is in the right range to carry out detection.

The features, objects and advantages of the plate according to the present invention will be more apparent from the following detailed description, to be read making reference to the figures of the accompanying sheets of illustrative drawings, in which:

Fig. 1 is a perspective view of the plate, complete with strap and tying ribbon;

Fig. 2 is a plan view of the plate of Fig. 1;

Figs. 3A and 3B are views of two kinds of room temperature indicators, which may be applied on the plate of the invention;

Fig. 4 is a perspective view of the plate with strap and central cord; and

Fig. 5 is a plan view of the plate of Fig. 4.

With reference now to the various figures of the accompanying drawings, the plate for thermographic detection according to present invention consists of a support 1 of flexible material, preferably anti-allergic plastic material, having two areas 2 and 3 of anatomic shape, comprising layers of thermosensitive liquid crystals, and more particularly the areas 2 and 3 have the lateral curved zones 4 allowing to effect the contemporaneous thermographic detection of the outer quadrants of both mammae.

As hereinbefore indicated, the liquid crystals applied on the areas 2 and 3 may comprise only one layer, generally formed by one type only of liquid crystals having a fixed colour changing temperature range, and in this case the plate will be preferably intended for professional use by a physician who will have at disposal a set of plates adapted for detection at different temperatures, or they comprise two or more superposed layers, so that the plate may be used with a much broader temperature range, and this type of plate is preferably intended for direct use by the individual patient.

Support 1 is also provided with two upper bores 5 for applying a strap 6 to be worn on the shoulders or on the neck, or two side bores 7 for applying strings or ribbons 8 to be tied on the back. The ribbons 8 may be joined by means of friction adhering bands made of a suitable rough material of the kind known on the market under the trade name "Velcro". The strap 6 and the ribbons 8 may also by provided together on the same plate.

Furthermore, the plate according to the present invention preferably has a room temperature indicator to check if this is falling in the range at which it is possible to carry out the thermographic detection in the best condition.

Such a room temperature indicator may consist of a strip 10 on which there is a little digital thermometer made of liquid crystals, showing the digits of the room temperature with intervals of 2° C, for instance from 16° C to 32° C, while near it there are boxes 11, 12 and 13 showing, when they become coloured, that room temperature is too low (16° - 20° C, box 11), ideal for the thermographic detection (20° - 24° C, box 12) or too high (above 24° C, box 13), respectively. A simpler type of indicator consists of a central box 14 showing, when coloured, that the temperature is correct, and two end boxes 15 and 16 which, when coloured, show that temperature is outside said correct range, i.e. either too high or too low. The liquid crystal room temperature indicators may be applied on the support 1 or inserted in it.

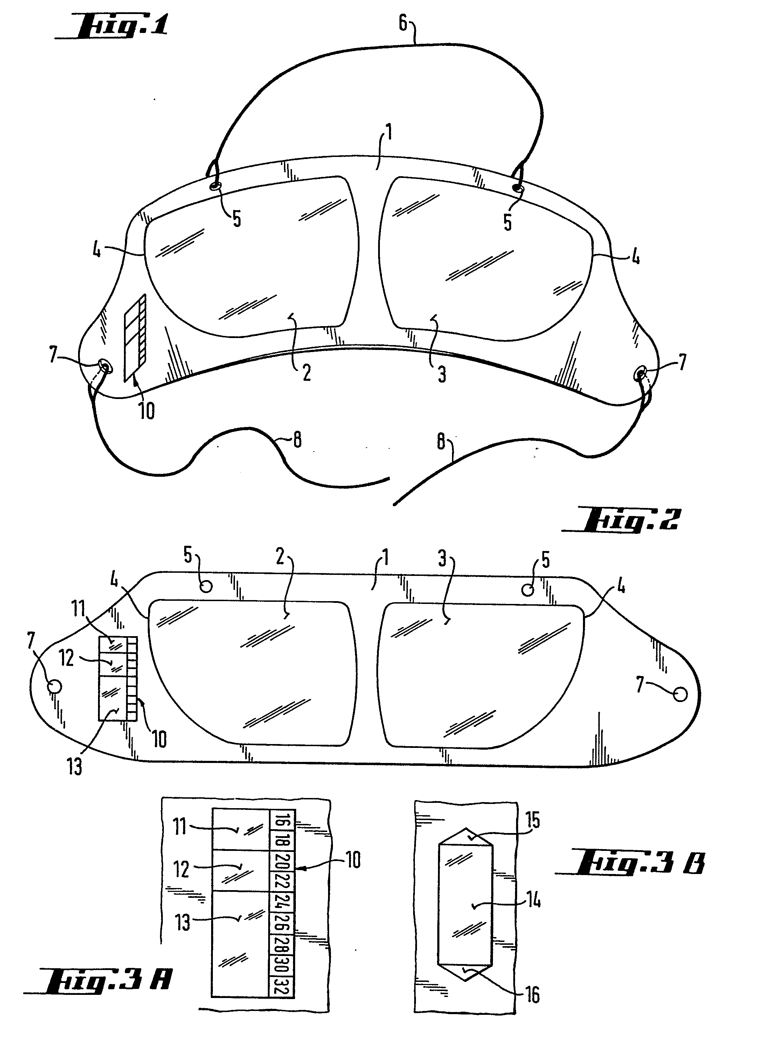

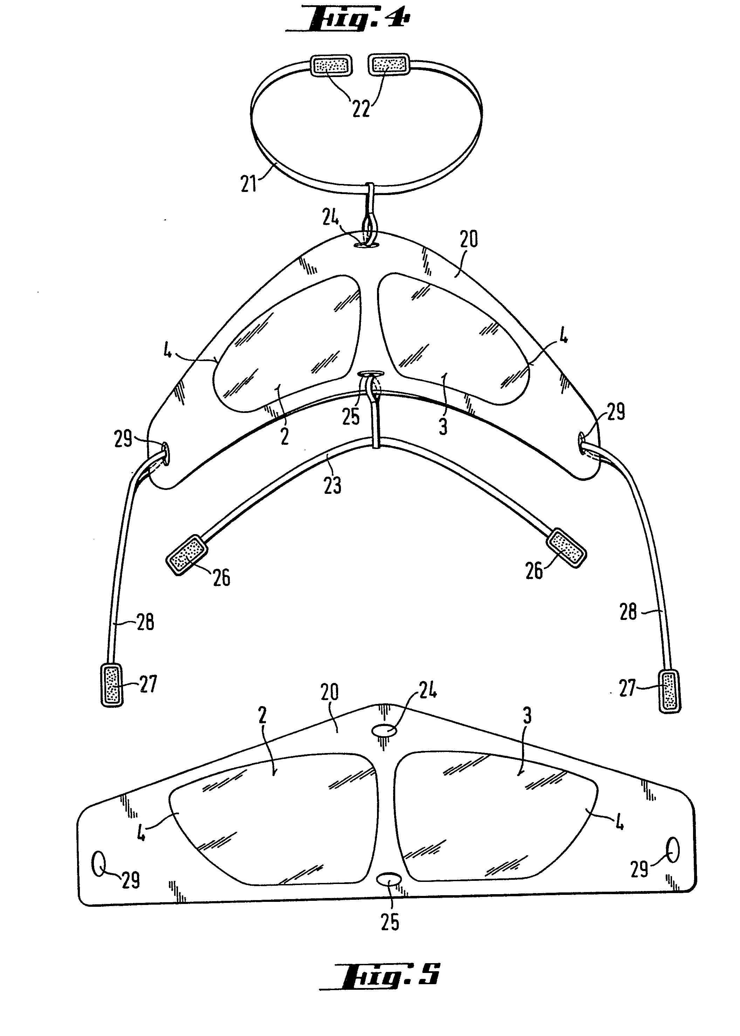

As the most important thing for a correct thermographic detection is that the plate adheres as precisely as possible to the breast, so that the plate follows at best the mammary contour, it is advisable that the plate is kept adhering to the body in the central groove between mammae, and this is effected in the plate 20 shown in Figs. 4 and 5, by a narrow strap 21 hung to the central upper eye 24 of the plate and to be tied on the neck by the Velcro hooks 22, and a lower strap 23 hung to the central lower eye 25 of the plate, also tied on the back with hooks 26, while adherence is completed by the usual central back hook 27 for the two ribbons 28 starting from the side eyes 29. The two liquid crystal areas 2 and 3 with the curved lateral zones 4 are substantially identical to those of the plate shown in Figs. 1 and 2. It is clear that also the plate of Figs. 4 and 5 may have the little room temperature indicator hereinbefore described.

Materials, dimensions and other numerical values previously set forth may of course be varied according to requirements, always respecting however the structural principles above indicated, as it is also pointed out in the appended claims.

0059328

CLAIMS

1) Microencapsulated liquid crystals plate for mammary thermographic detection, characterized by the fact of the comprising a flexible support (1) having two areas (2, 3) of anatomic shape, provided with layers of thermosensitive liquid crystals, and contemporaneously covering all quadrants of the breast, including the outer ones (4).

2) Plate according to Claim 1, characterized by the fact of having two or more superposed layers of liquid crystals, changing colour at different temperatures, so as to allow a broader range of detection temperatures.

3) Plate according to Claim 1, characterized by the fact of having only one layer of liquid crystals, being part of a set of plates, each plate to be optimally used at a different temperature range.

4) Plate according to Claim 1, characterized by the fact of having a strap (6) to be worn on the shoulders or on the neck and/or a ribbon (8) starting from the two lateral plate ends, which may be tied on the back like in brassières.

5) Plate according to Claim 1, characterized by the fact of having an upper strap (21) hung at the plate center (24) and a cord (23) which keeps the central lower part of said plate adhering to the body and is tied on the back.

6) Plate according to Claims 4 and/or 5, characterized by the fact that the closure of the tying members around the body is effected by hooks made of rough material such as that known under the trade name "Velcro".

7) Plate according to Claim 1, characterized by the fact of having a room temperature indicator (10; 14, 15, 16), also made of liquid crystals, applied on the support (1) to check if the room temperature is in the correct range for effecting detection.

8) Plate according to Claim 7, characterized by the fact that the room temperature indicator (10) is a little digital liquid crystal thermometer, near which another

0059328

liquid crystal indicator may be arranged, divided into boxes (11, 12, 13), each box showing, when coloured, if the room temperature indicated by the first indicator (10) is too low, correct or too high, respectively.

Fig.1

Fig.2

Fig.3 A

Fig.3 B

**Fig. 4**

**Fig. 5**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US-E- 30 446 (P.H.MEYERS et al./E-Z-EM Co., INC.) *Abstract; column 5, lines 19-40; column 6, lines 7-48; column 7, lines 11-17 and lines 55-58; figures 1-4* | 1,3-6 | A 61 B 5/00 G 01 K 11/16 |
| Y | US-A-3 847 139 (E.FLAM) *Abstract; column 3, lines 15-62; column 4, lines 46-52; column 5, line 54 - column 6, line 6; column 6, line 40 - column 7, line 22; figures 1-6* | 1,2,4-6 | |
| A | US-A-3 830 224 (R.VANZETTI et al./VENZETTI INFRARED & COMPUTER SYSTEMS, INC.) *Abstract; column 3, lines 31-44; column 4, lines 35-50; figures 2,7,8* | 1,2,4 | TECHNICAL FIELDS SEARCHED (Int. Cl. 3) |
| A | GB-A-2 023 288 (ARDEN INDUSTRIES, INC.) *Abstract; page 2, lines 46-91; page 3, lines 37-50; figures 1-4* | 1,8 | A 61 B 5/00 G 01 K 11/16 |

The present search report has been drawn up for all claims

| Place of search THE HAGUE | Date of completion of the search 08-06-1982 | Examiner RIEB D.K. |
|---|---|---|